# EUROPEAN PATENT SPECIFICATION

(11) **EP 3 630 128 B1**
(45) Date of publication and mention of the grant of the patent: **17.04.2024**
(21) Application number: 17737636.5
(22) Date of filing: 22.05.2017
(51) Int. Cl.: A61K 35/00, A61K 35/747, C12N 1/20, A23L 33/00, A23L 33/135, A23C 9/123, A61P 1/16, A61P 31/04

(54) **COMPOSITIONS AND METHODS FOR DECREASING A POPULATION OF BILOPHILA WADSWORTHIA OR INHIBITING THE GROWTH THEREOF**
ZUSAMMENSETZUNGEN UND VERFAHREN ZUR VERRINGERUNG EINER POPULATION VON BILOPHILA WADSWORTHIA ODER ZUR HEMMUNG DES WACHSTUMS DAVON
COMPOSITIONS ET PROCÉDÉS POUR DIMINUER UNE POPULATION DE BILOPHILA WADSWORTHIA OU INHIBER LA CROISSANCE DE CELLE-CI

(43) Date of publication of application: 08.04.2020
(73) Proprietor: Compagnie Gervais Danone, 75009 Paris (FR); Institut national de recherche pour l'agriculture, l'alimentation et l'environnement, 75007 Paris (FR)
(72) Inventor: SOKOL, Harry, 75015 Paris (FR); LANGELLA, Philippe, 78140 Velizy (FR); NATIVIDAD, Jane, 75015 Paris (FR); VEIGA, Patrick, 92800 Puteaux (FR); VAN HYLCKAMA VLIEG, Johan, 78450 Chavenay (FR)
(74) Representative: Gevers & Orès
(86) International application number: PCT/IB2017/000766
(87) International publication number: WO 2018/215809

(56) References cited:
- US-A1- 2010 028 449
- US-A1- 2015 297 646
- Y. NI ET AL: "A metagenomic study of the preventive effect of Lactobacillus rhamnosus GG on intestinal polyp formation in Apc Min/+ mice", JOURNAL OF APPLIED MICROBIOLOGY., vol. 122, no. 3, 30 January 2017 (2017-01-30), pages 770-784, XP055439294, GB ISSN: 1364-5072, DOI: 10.1111/jam.13386
- CHENG-CHIH TSAI ET AL: "Cholesterol-Lowering Potentials of Lactic Acid Bacteria Based on Bile-Salt Hydrolase Activity and Effect of Potent Strains on Cholesterol Metabolism In Vitro and In Vivo", THE SCIENTIFIC WORLD JOURNAL, vol. 2014, 1 January 2014 (2014-01-01), pages 1-10, XP055439712, ISSN: 2356-6140, DOI: 10.1155/2014/690752

## Description

### Field of the invention

The present invention relates to compositions for use in treating bile acid dysmetabolism.

### Technical background

Over the last three decades, the prevalence of obesity as well associated metabolic complications such as type 2 diabetes and non-associated fatty liver diseases has significantly increased. The increasing epidemic have been linked to changes in lifestyle choices, specifically, the increased consumption of a "Western-style diet", that is rich in saturated fat and sugar.

However, within the intersection of diet and metabolic health, large bodies of research also implicate the intestinal microbiota in modulating these diseases.

As such, the observation that germ-free mice are protected from high-saturated fat diet (HFD)-induced obesity as well as showed improved glycemic control and hepatic phenotype when compared with colonized counterparts highlights the role of gut microbiota in mediating the impact of diet on the host metabolic status. The importance of these initial findings are further supported by epidemiological studies showing that obesity is associated with gut microbiota alterations, and human obese microbiota phenotype can be conferred to mice by transplanting obese microbiota into germ-free mice.

Additionally, different diets have been shown to rapidly and reproducibly alter both the composition and function of the microbiota. Certain groups or strains of bacteria have been associated with different types of diets. For instance, *Bilophila wadsworthia* has been associated with animal based diet as well as diets rich in fats.

Thus, gut microbiota alterations seem to be a hallmark of metabolic impairment and although causal relationships that underlie this process are complex and are not completely understood, there have been attempts to reprogram the composition and function of the microbiota, with the view of improving the health status of individuals.

In this regard, Odamaki et al. (2016) Beneficial Microbes 7:473-484 report that the intake of yoghurt supplemented with *Bifidobacterium longum* played a role in maintaining a normal microbiota composition, in particular by preventing an increase in the intestinal population of *Bilophila wadsworthia,* in individuals submitted to a meat-based (*i.e*. animal-based) diet. However, these authors do not show that the control of the population of *Bilophila wadsworthia* by *Bifidobacterium longum* is associated to health benefits for the individuals. In addition, there is always a need for alternative agents for controlling bacterial populations.
Ni et al. (2017) Journal of Applied Microbiology 122:3, 770-784 report that the intestinal abundance of *B. wadsworthia* was diminished in a group of individuals treated with *Lactobacillus rhamnosus* GG.
Cheng-Chih et al. (2014) The Scientific World Journal, 1-10 report that *L. rhamnosus* showed bile-salt hydrolase activity *in vitro.*
US 2010/028449 A1 discloses lactobacilli having bile salt hydrolase activity.
US 2015/297646 A1 discloses *L. rhamnosus* CNCM I-3690 for use in the treatment of metabolic disorders.

### Summary of the invention

The present invention follows from the unexpected finding that *Lactobacillus* bacterium, have the ability to decrease or inhibit *Bilophila wadsworthia* in-vivo, thus leading to improvement of associated metabolic impairments and host dysfunctions including but not limited to bile acid.

The invention is as defined in claims 1 to 6.

The present invention relates to a composition comprising, or conditioned by, at least one *Lactobacillus* bacterium for use in reducing bile acids in an individual.

### Detailed description of the invention

As intended herein "at least one *Lactobacillus* bacterium or a composition comprising thereof" shall be taken to mean a composition comprising at least one *Lactobacillus* bacterium.

As intended herein a composition "conditioned thereby" means that that the composition comprises secretions from lactic acid bacteria according to the invention. By way of example, the composition conditioned by lactic acid bacteria according to the invention may comprise the supernatant of a culture of lactic acid bacteria or it can be a dairy product fermented by lactic acid bacteria from which the lactic acid bacteria have been removed.

As used herein the term "supernatant" shall be taken to mean the culture medium in which bacteria has been grown under conditions suitable for growth. The culture media may be separated from the bacterial cells and fragments thereof by means of centrifugation.

As used herein the term "stable composition" shall be taken to mean a composition that does not present sedimentation and/or serum separation.

As used herein the term "x% (w/w)" is equivalent to "x g per 100 g".

As used herein the term "spoonable" shall be taken to mean a solid or semi-solid that may be consumed by means of a spoon or other utensil.

As used herein the term "fermentation" shall be taken to mean the metabolism of a substance by bacteria, yeasts, or other microorganisms.

As used herein the term "cfu" or "CFU" shall be taken to be an abbreviation of the term "colony forming unit".

As used herein the term "overweight" shall be taken to mean a BMI (body mass index) of 25-30.

As used herein the term "obese" shall be taken to mean a BMI (body mass index) over 30.

As used herein the term "CNCM I-" followed by a 4 digit number shall be taken to refer to a strain deposited at the Collection Nationale de Cultures de Microorganismes (CNCM) 25 rue du Docteur Roux, Paris, France under the Budapest Treaty with an accession number corresponding to said 4 digit number, e.g. CNCM I-3690.

As used herein reference to a bacterial strain or species shall be taken to include functionally equivalent bacteria derived therefrom such as but not limited to mutants, variants or genetically transformed bacteria. These mutants or genetically transformed strains can be strains wherein one or more endogenous gene(s) of the parent strain has (have) been mutated, for instance to modify some of their metabolic properties (*e.g*., their ability to ferment sugars, their resistance to acidity, their survival to transport in the gastrointestinal tract, their post-acidification properties or their metabolite production). They can also be strains resulting from the genetic transformation of the parent strain to add one or more gene(s) of interest, for instance in order to give to said genetically transformed strains additional physiological features, or to allow them to express proteins of therapeutic or prophylactic interest that one wishes to administer through said strains. These mutants or genetically transformed strains can be obtained from the parent strain by means of conventional techniques for random or site-directed mutagenesis and genetic transformation of bacteria, or by means of the technique known as "genome shuffling". In the present text, strains, mutants and variants derived from a parent species or strain will be considered as being encompassed by reference to said parent species or strain, e.g. the phrases "*L. rhamnosus"* and "strain CNCM I-3690" shall be taken to include strains, mutants and variants derived therefrom.

Accordingly, as used herein reference to a bacterial strain specified by an accession or deposit number shall be taken to encompass variants thereof having at least 80 % identity with the 16S rRNA sequence of said specified strain, preferably at least 85 % identity, more preferably at least 90 % identity, further preferably at least 95 % identity (see: Stackebrandt & Goebel, 1994, Int. J. Syst. Bacteriol. 44:846-849). In a particularly preferred embodiment, said variant has at least 97 % identity with the 16S rRNA sequence of said specified strain, more preferably at least 98 % identity, more preferably at least 99 % identity.

The lactic acid bacteria comprised in the composition according to the invention may be living or dead bacteria. Preferably, at least some of the lactic acid bacteria comprised in the composition according to the invention are living bacteria. More preferably, the composition according to the invention comprises from 10⁵ to 10¹⁰ colony forming unit of lactic acid bacteria per gram of composition (CFU/g), more preferably at least 10⁷, 10⁸ 10⁹ colony forming unit of lactic acid bacteria per gram of composition (CFU/g).

The composition according to the invention is preferably a food or nutritional composition, *i.e.* a food product, more preferably a dairy product, and most preferably a fermented dairy product, in particular fermented by the lactic acid bacteria according to the invention.

As intended herein a fermented dairy product is the fermentation product of a milk-based composition by a starter culture of fermenting microorganisms, in particular bacteria, more particularly lactic acid bacteria. The fermented dairy product according to the invention can thus be a fermented milk, a yoghurt, in particular a set, stirred or drink yogurt, or a fresh cheese such as a white cheese or a petit-Suisse. It can be also a strained fermented dairy product such as a strained yoghurt also called concentrated yoghurt or Greek-style yoghurt.

The terms "fermented milk" and "yogurt" or "yoghurt" are given their usual meanings in the field of the dairy industry, that is, products destined for human consumption and originating from acidifying lactic fermentation of a milk substrate. These products can contain secondary ingredients such as fruits, vegetables, sugar, etc.

The expression "fermented milk" is thus reserved in the present application for a dairy product prepared with a milk substrate which has undergone treatment at least equivalent to pasteurization, seeded with microorganisms belonging to the characteristic species or species of each product.

The term "yogurt" or "yoghurt" is reserved for fermented milk obtained, according to local usage, by the development of specific thermophilic lactic bacteria known as *Lactobacillus delbrueckii* subsp*. bulgaricus* and *Streptococcus thermophilus*, which must be in the living state in the finished product, at a minimum rate. In certain countries, regulations require the addition of other lactic bacteria to the production of yoghurt, and especially the additional use of strains of *Lactobacillus rhamnosus* and/or *Lactobacillus acidophilus* and /or *Lactobacillus casei.* These additional lactic strains are intended to impart various properties to the finished product, such as that of favouring equilibrium of intestinal flora or modulating the immune system.

In practice, the expression "fermented milk" is therefore generally used to designate fermented milks other than yogurts. It can also, according to country, be known by names as diverse as, for example, "Kefir", "Kumtss", "Lassi", "Dahi", "Leben", "Filmjolk", "Villi", "Acidophilus milk".

The term "white cheese" or "petit-Suisse" is, in the present application, reserved for unrefined non-salty cheese, which has undergone fermentation by lactic acid bacteria only (and no fermentation other than lactic fermentation).

The fermented dairy product can be made from whole milk and/or wholly or partly skimmed milk, which can be used in a powder form which can be reconstituted by addition of water. Other milk components can be added such as cream, casein, caseinate (for example calcium or sodium caseinate), whey proteins notably in the form of a concentrate (WPC), milk proteins notably in the form of a concentrate (MPC), milk protein hydrolysates, and mixtures thereof.

As intended herein, a "lactic acid bacterium" is a Gram-positive, acid-tolerant, generally non-sporulating and non-respiring, either rod- or cocci-shaped bacterium that is able to ferment sugars in lactic acid.

As intended herein "increasing or maintaining" a bacterial population, in particular an intestinal population thereof, means protecting, favoring or stimulating the growth of said bacteria so that the count of said bacteria, or the relative number of said bacteria with respect to other bacteria, is maintained or increases.

As intended herein "decreasing" a bacterial population, in particular an intestinal population thereof, means reducing the growth of said bacteria so that the count of said bacteria, or the relative number of said bacteria with respect to other bacteria, is decreased.

As intended herein "inhibiting" a bacterial population, in particular an intestinal population thereof, means preventing the growth of said bacteria so that the count of said bacteria, or the relative number of said bacteria with respect to other bacteria, is not increased.

As intended herein an intestinal population of *Bilophila wadsworthia* relates to the *Bilophila wadsworthia* bacteria present in the intestine or gut, in particular the colon, of an individual.

Determining the quantity of *Bilophila wadsworthia* bacteria in a sample can be performed by numerous methods well known to one of skill in the art. Determining the intestinal quantity of *Bilophila wadsworthia* bacteria in an individual can be performed by culturing stool samples of the individual.

Preferably, the individual according to the invention is a human individual. In one embodiment, the individual according to the invention is a healthy individual or an individual who does not suffer from intestinal diseases or diseases of the gastrointestinal tact

As intended herein "non-therapeutic" means that the individual receiving or consuming the composition according to the invention is not treated for a disease by the composition. In other words, within the frame of the non-therapeutic uses and methods according to the invention, the composition according to the invention is neither a medicament nor a pharmaceutical composition.
The herein described invention pertains to at least one *Lactobacillus* bacterium for uses as described below, and in additional embodiments to compositions and products. The lactic acid bacterium is *Lactobacillus rhamnosus* CNCM I-3690.The strain *Lactobacillus rhamnosus* CNCM I-3690 has been deposited at the Collection Nationale de Cultures de Microorganismes (CNCM) (Institut Pasteur, 25 Rue du Docteur Roux, Paris, France) under the Budapest Treaty on Nov. 9, 2006, under number I-3690.

### Compositions

The present invention also relates to a composition comprising, or conditioned by, at least one *Lactobacillus* bacterium for use in reducing bile acids.

The composition for use according to embodiments of the invention is suitable for consumption or ingestion, preferably by oral means. Accordingly the composition comprises or consists of comestible matter. It is particularly preferred that the compositions of embodiments of the invention are substantially free of pathogenic or toxicogenic matter. The composition according to embodiments of the invention may be a medicament or pharmaceutical composition. In a particularly preferred embodiment the composition according to the invention may be a non-therapeutic composition, preferably a nutraceutical composition, a nutritional composition and/or a food composition.

In one embodiment said bile acids are serum or cecal bile acids. It is particularly preferred that said bile acids are tauro-conjugated, in a further preferred embodiment said bile acids are selected from the group consisting of taurocholic acid (TCA) and UDCA.

In one embodiment said bile acid dysmetabolism is a disorder associated with or caused by intestinal *Bilophila wadsworthia.*

Preferably, the composition comprises at least 10⁶, more preferably at least 10⁷ and most preferably at least 10⁸ colony forming unit (CFU) of lactic acid bacteria, preferably *L. rhamnosus,* according to embodiments of the invention per gram (g) of composition according to embodiments of the invention. Preferably also the composition according to embodiments of the invention comprises at least about 10¹¹, more preferably at least 10¹⁰ and most preferably at least 10⁹ colony forming unit (CFU) of lactic acid bacteria, preferably *Lactobacillus rhamnosus,* according to embodiments of the invention per gram (g) of composition according to embodiments of the invention. In a further embodiment the lactic acid bacteria is *Lactobacillus* rhamnosus CNCM I-3690.

In embodiments, the composition comprises 10⁶ to 10¹¹ colony forming unit (CFU) lactic acid bacteria, preferably *Lactobacillus rhamnosus,* according to embodiments of the invention per gram (g) of composition according to embodiments of the invention. In embodiments, the composition comprises 10⁷ to 10¹¹ colony forming unit (CFU) of lactic acid bacteria, preferably *Lactobacillus rhamnosus,* according to embodiments of the invention per gram (g) of composition according to embodiments of the invention. In embodiments, the composition comprises 10⁸ to 10¹¹ colony forming unit (CFU) of lactic acid bacteria, preferably *Lactobacillus rhamnosus,* according to embodiments of the invention per gram (g) of composition according to embodiments of the invention. In embodiments, the composition comprises 10⁹ to 10¹¹ colony forming unit (CFU) of lactic acid bacteria, preferably *Lactobacillus rhamnosus,* according to embodiments of the invention per gram (g) of composition according to embodiments of the invention. In embodiments, the composition comprises 10¹⁰ to 10¹¹ colony forming unit (CFU) of lactic acid bacteria, preferably *Lactobacillus rhamnosus,* according to embodiments of the invention per gram (g) of composition according to embodiments of the invention.

In embodiments, the composition comprises 10⁶ to 10¹⁰ colony forming unit (CFU) of lactic acid bacteria, preferably *Lactobacillus rhamnosus,* according to embodiments of the invention per gram (g) of composition according to embodiments of the invention. In embodiments, the composition comprises 10⁶ to 10⁹ colony forming unit (CFU) of lactic acid bacteria, preferably *Lactobacillus rhamnosus,* according to embodiments of the invention per gram (g) of composition according to embodiments of the invention. In embodiments, the composition comprises 10⁶ to 10⁸ colony forming unit (CFU) of lactic acid bacteria, preferably *Lactobacillus rhamnosus,* according to embodiments of the invention per gram (g) of composition according to embodiments of the invention. In embodiments, the composition comprises 10⁶ to 10⁷ colony forming unit (CFU) of lactic acid bacteria, preferably *Lactobacillus rhamnosus,* according to embodiments of the invention per gram (g) of composition according to embodiments of the invention. It is particularly preferred that the lactic acid bacteria is *Lactobacillus rhamnosus* CNCM I-3690.

Preferably the composition suitable for the uses and methods of embodiments of the invention comprises milk, more preferably fermented milk. Preferably the composition comprises at least about 30 % (w/w) milk, more preferably at least about 50% (w/w) milk and even more preferably at least about 70% (w/w) milk. In embodiments, the composition comprises at 30 % to 100% (w/w) milk. In embodiments, the composition comprises 50% to 100% (w/w) milk. In embodiments, the composition comprises 70% to 100% (w/w) milk. Preferably said milk is vegetal and/or animal milk, more preferably soya, almond, oat, hemp, spelt, coconut, rice, goat, ewe, camel, mare or cow milk, and most preferably to cow milk. Preferably said milk(s) are heat-treated, typically pasteurized, to ensure sterility. Preferably said heat treatment is carried out prior to the preparation of the fermented milk composition.

Preferably said milk comprises one or more of skimmed, partially-skimmed or non-skimmed milk. Preferably said milk or milks may be in liquid, powdered and/or concentrated form. In one embodiment said milk further comprises milk components preferably selected from the group consisting of cream, casein, caseinate (for example calcium or sodium caseinate), whey proteins notably in the form of a concentrate (WPC), milk proteins notably in the form of a concentrate (MPC), milk protein hydrolysates, and mixtures thereof. In one embodiment said mixture further comprises plant and/or fruit juices. In one embodiment said milk or milks may be enriched or fortified with further milk components or other nutrients such as but not limited to vitamins, minerals, trace elements or other micronutrients.

Preferably the composition comprises above about 0.3 g per 100 g by weight free lactic acid, more preferably above about 0.7 g or 0.6 g per 100 g by weight free lactic acid. In embodiments, the composition comprises 0.3 g to 0.7 grams per 100 g by weight free lactic acid.

Preferably the composition comprises a protein content at least equivalent to that of the milk or milks from which it is derived, preferably at least about 2.5%, more preferably at least about 3% or 3.5% (w/w). Preferably the composition has a pH equal to or lower than 5, preferably between about 3 and about 4.5 and more preferably between about 3.5 and about 4.5.

Preferably the composition has a viscosity lower than 200 mPa.s, more preferably lower than 100 mPa.s and most preferably lower that 60 mPa.s, at 10°C, at a shear rate of 64 s⁻¹. In embodiments, the composition has a viscosity range of 1 to 200 mPa.s, 1 to 100 mPa.s, or 1 to 60 mPa.s, at 10°C, at a shear rate of 64 s⁻¹. In embodiments, the composition has a viscosity range of 10 to 200 mPa.s, 10 to 100 mPa.s, or 10 to 60 mPa.s, at 10°C, at a shear rate of 64 s⁻¹. In embodiments, the composition has a viscosity range of 30 to 200 mPa.s, 30 to 100 mPa.s, or 30 to 60 mPa.s, at 10°C, at a shear rate of 64 s⁻¹.

The composition according to embodiments of the invention is preferably a product selected from the group comprising yogurt, set yogurt, stirred yogurt, pourable yogurt, yogurt drink, frozen yogurt, kefir, buttermilk, quark, sour cream, fresh cheese and cheese. In one embodiment the composition according to embodiments of the invention is a drinkable composition, more preferably a fermented milk drink such as but not limited to a yogurt drink, kefir etc.. In an alternative embodiment the composition according to embodiments of the invention is a composition that is spoonable such as a set or stirred yogurt or equivalent thereof.

Preferably the composition, according to embodiments of the invention, may be stored, transported and/or distributed at a temperature of from 1°C to 10°C for at least about 30 days, at least about 60 days or at least about 90 days from packaging and remain suitable for consumption.

Preferably, the composition is a packaged product that comprises at least 10⁶, more preferably at least 10⁷ and most preferably at least 10⁸ colony forming unit (CFU) of lactic acid bacteria, preferably *Lactobacillus rhamnosus,* according to embodiments of the invention per gram (g) of composition according to embodiments of the invention subsequent to storage, transport and/or distribution at a temperature of from 1°C to 10°C for at least about 30 days, at least about 60 days or at least about 90 days from packaging. It is particularly preferred that the lactic acid bacteria is *Lactobacillus rhamnosus* CNCM I-3690.

In embodiments, the composition is a packaged product that comprises 10⁶ to 10⁸ or 10⁶ to 10⁷ colony forming unit (CFU) of lactic acid bacteria, preferably *Lactobacillus rhamnosus,* according to embodiments of the invention per gram (g) of composition according to embodiments of the invention subsequent to storage, transport and/or distribution at a temperature of from 1°C to 10°C for at least about 30 days, at least about 60 days or at least about 90 days from packaging. It is particularly preferred that the lactic acid bacteria is *Lactobacillus rhamnosus* CNCM I-3690.

According to a further embodiment, the composition further comprises an intermediate preparation comprising a preparation of fruits and/or cereals and/or additives such as flavorings and/or colorings. Said intermediate preparation can in particular contain thickeners (soluble and insoluble fibres, alginates, carragheenans, xanthan gum, pectin, starch, in particular gelatinized, gelan gum, cellulose and its derivatives, guar and carob gum, inulin) or sweeteners (aspartame, acesulphame K, saccharine, sucralose, cyclamate) or preservatives. Examples of flavorings are: apple, orange, strawberry, kiwi fruit, cocoa flavoring etc. Examples of colorings are: beta-carotene, carmine, cochineal red. Moreover, the preparation of the abovementioned fruits can comprise fruits which are whole or in pieces or in jelly or in jam, making it possible for example to obtain fruit yogurts.

Preferably the composition according to embodiments of the invention comprises up to about 30% (w/w) of said intermediate preparation, e.g. up to about 10%, 15%, 20%, 25% (w/w). In one embodiment, the composition according to embodiments of the invention comprise 1% to 30% (w/w) of said intermediate preparation. In alternative embodiments, the composition according to embodiments of the invention comprise 1% to 25% (w/w) of said intermediate preparation. In further alternative embodiments, the composition according to embodiments of the invention comprise 1% to 20% (w/w) of said intermediate preparation. In additional embodiments, the composition according to embodiments of the invention comprise 1% to 15% (w/w) of said intermediate preparation. In further additional embodiments, the composition according to embodiments of the invention comprise 1% to 10% (w/w) of said intermediate preparation.

Preferably the composition, according to embodiments of the invention is provided in a sealed or sealable container containing about 50 g, 60 g, 70 g, 75 g, 80 g, 85 g, 90 g, 95 g, 100 g, 105 g, 110 g, 115 g, 120 g, 125 g, 130 g, 135 g, 140 g, 145 g, 150 g, 200 g, 300 g, 320 g or 500 g or about 1 oz, 2 oz, 3 oz, 4 oz, 5 oz, 6 oz or 12 oz product by weight.

In embodiments, the composition, according to embodiments of the invention is provided in a sealed or sealable container containing about 50 g to 500 g, 60 g to 500 g, 70 g to 500 g, 75 g to 500 g, 80 g to 500 g , 85 g to 500 g, 90 g to 500 g, 95 g to 500 g, 100 g to 500 g, 105 g to 500 g, 110 g to 500 g, 115 g to 500 g, 120 g to 500 g, 125 g to 500 g, 130 g to 500 g, 135 g to 500 g, 140 g to 500 g, 145 g to 500 g, 150 g to 500 g, 200 g to 500 g, 300 g to 500 g, 320 g to 500 g or 500 g product by weight. In embodiments, the composition, according to embodiments of the invention is provided in a sealed or sealable container containing about 1 oz to 12 oz, 2 oz to 12 oz, 3 oz to 12 oz, 4 oz to 12 oz, 5 oz to 12 oz, 6 oz to 12 oz or 12 oz product by weight.

### Methods for the preparation of fermented milk products

Methods for the preparation of fermented milk products, such as yogurts or equivalents thereof, are well-known in the art and are disclosed for illustration purposes only.

Preferably fermented milk products are prepared using milk that has been subjected to heat treatment at least equivalent to pasteurization. Preferably said heat treatment is carried out prior to the preparation of the composition.

Typically, milk is pasteurized by means of the following successive steps:
1) standardization of fatty substances of the raw material so as to obtain a standardized substance,
2) enrichment with dried matter of the standardized substance obtained in the preceding stage, so as to obtain an enriched substance,
3) preheating of the enriched substance obtained in the preceding stage, so as to obtain a starting substance,
4) pasteurization and holding of the starting substance obtained in the preceding stage, so as to obtain a pasteurized and held substance,
5) an optional stage of homogenization of the pasteurized and held substance obtained in the preceding stage, so as to obtain a pasteurized, held and optionally homogenized substance,
6) initial cooling of the pasteurized, held and optionally homogenized substance obtained in the preceding stage, so as to obtain a pasteurized starting substance that has been held, optionally homogenized, and cooled down.

As used herein "standardization of fatty substances" is taken to mean a stage of bringing the quantity of fats present in the starting substance to a pre-determined level. Enrichment with dried matter involves the addition of proteins and fatty substance in order to modify curd firmness.

As used herein "holding" is taken to mean a rapid thermalization of the milk and makes it possible to destroy the vegetative microbial flora, including pathogenic forms. Its typical duration is from 4 to 10 minutes, in particular from 5 to 8 minutes, and in particular approximately 6 minutes.

As used herein "homogenization" is taken to mean the dispersion of the fatty substances in the milk-type substance into small fat globules. The homogenization is carried out for example at a pressure of 100 to 280 bars, in particular 100 to 250 bars, in particular 100 to 200 bars, in particular approximately 200 bars. This homogenization stage is purely optional. It is in particular absent from the production process of products with 0% fatty substances.

Typically a fermented milk product is prepared by culture of milks at a suitable temperature with suitable microorganisms to provide a reduction in pH, preferably to a pH equal to or lower than 5, preferably between about 3 and 4.5; more preferably between about 3.5 and about 4.5. The pH can be adjusted by controlling the fermentation by the microorganism and stopping it when appropriate, for example by cooling.

The selection of suitable lactic acid bacteria strains is within the scope of the skilled person and is typically a thermophillic lactic acid bacteria. Examples of lactic acid bacteria that can be used include but are not limited to *Lactobacilli* (for example *Lactobacillus acidophilus*, *Lactobacillus buchneri, Lactobacillus delbruckei,* in particular *L. delbruckeisupsb. bulgaricus* or *lactis, Lactobacillus casei, Lactobacillus plantarum, Lactobacillus reuteri, Lactobacillus johnsonii, Lactobacillus helveticus, Lactobacillus brevis, Lactobacillus rhamnosus*); *Streptococci* (for example *Streptococcus thermophilus*); *Lactococci* (for example *Lactococcus lactis*, typically *Lactococcus lactis subsp. lactis* or *Lactococcus lactis subsp. cremoris*)*.* Typically a mixture or association of a plurality of species of lactic acid bacteria may be used, typically a mixture or association of *Lactobacillus* and *Streptococcus.* For the preparation of yogurt this typically includes *Lactobacillus bulgaricus* (also referred to as *Lactobacillus delbruckei subsp. bulgaricus*) and *Streptococcus thermophilus*, optionally with additional microorganisms such as but not limited to probiotic species or other species that may provide desirable organoleptic or other qualities to the composition, e.g. *Lactococcus lactis.*

Suitable temperatures for milk fermentation are typically about 36°C to about 44°C and the temperature is maintained for an incubation time sufficient to provide the desired reduction in pH.

For the preparation of a fermented milk product the temperature at the start of fermentation is typically about 36°C to about 43°C, in particular about 37°C to about 40°C, the temperature at the end of fermentation is typically about 37°C to about 44°C, in particular about 38°C to about 41°C. The fermentation time is typically about 6 to about 11 hours.

Subsequent to the fermentation the fermented milk is cooled. Optionally a stage of intermediate cooling of the fermented milk may be performed to provide a pre-cooled fermented milk having a temperature of between about 22°C and about 4°C. Typically the intermediate cooling time is about 1 hour to about 4 hours, in particular about 1 hour 30 minutes to about 2 hours. The pre-cooled fermented milk is typically stored for up to 40 hours or less.

Preferably a stage of final cooling of the fermented milk is performed such that the temperature at the start of the final cooling is less than about 22°C and the temperature at the end of the final cooling is about 4°C to about 10°C. The cooled product may then be stored, transported and/or distributed at a temperature from about 1°C to about 10°C for at least about 30 days, at least about 60 days or at least about 90 days.

According to a further illustration, the process for the preparation of a fermented milk product as defined above optionally comprises a stage of stirring at a pressure of at least 20 bars, or performing a dynamic smoothing, to obtain a composition having the desired viscosity, typically a viscosity of up to 20 mPa.s. Stirring or dynamic smoothing operations provide some shear to composition that typically allow a reduction in viscosity. Such operations are known by the one skilled in the art, and can be operated with conventional appropriate equipment. This stage is typically performed at cold temperature, for example at a temperature of form 1 °C to 20°C. Without intending to be bound to any theory, it is believed that applying some shear at cold temperature, typically by stirring at high pressure or by performing a dynamic smoothing, can lead to a fluid gel formation within the composition, that provides improved stability even at a low viscosity of up to 20 mPa.s.

According to a further embodiment, the process for the preparation of a fermented milk product as defined above optionally comprises a stage of addition of an intermediate preparation prior or subsequent to fermentation, said intermediate preparation comprising a preparation of fruits and/or cereals and/or additives such as flavorings and/or colorings.

The invention will be further illustrated by the following non-limiting Figures and Example.

### Description of the figures

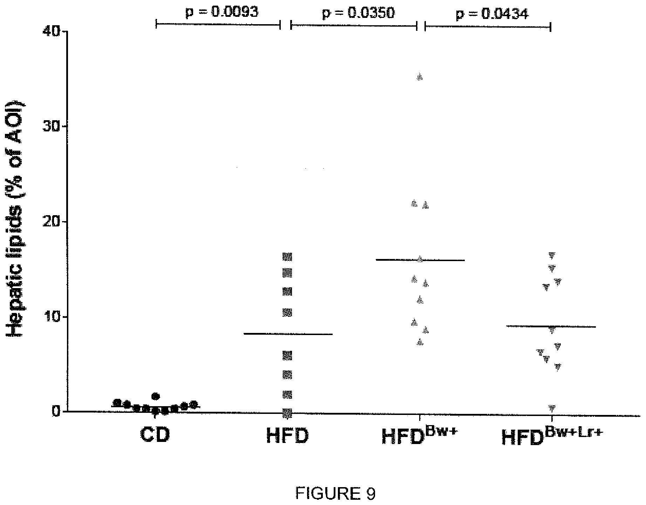

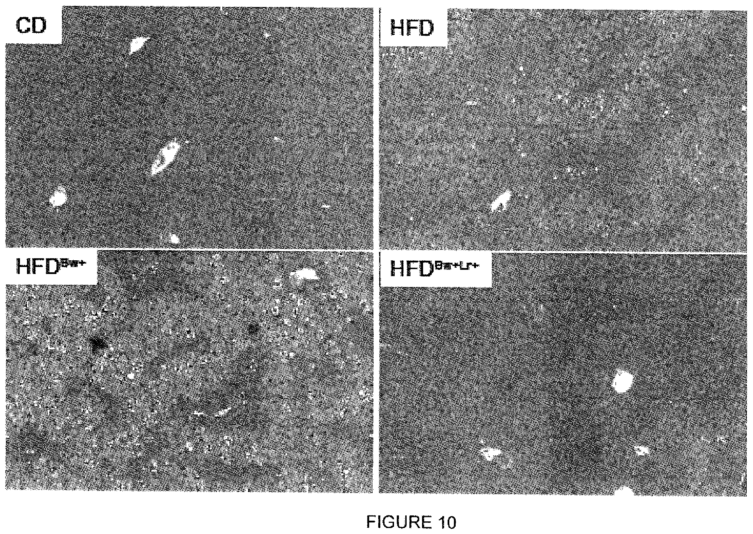

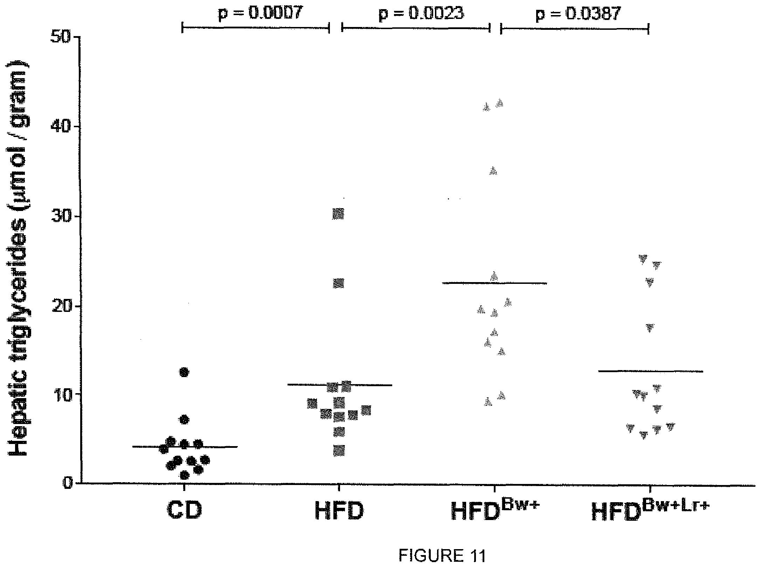

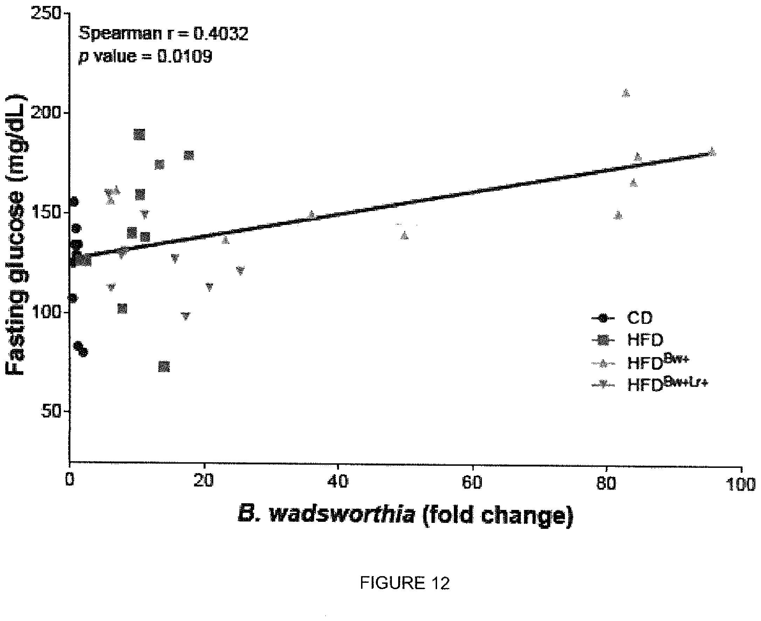

Figures 1 -12. *B. wadsworthia* synergizes with HFD to trigger stronger metabolic impairment.
Figure 1: Fold change of *B. wadsworthia* relative from day 0 in mice fed with control diet (CD) or high-fat diet (HFD) and inoculated with *B. wadsworthia* (Bw+) and treated with *L. rhamnosus* CNCM I-3690 (Lr+) (n=16-28/group).
Figure 2: *B. wadsworthia* load in small intestinal (SI), fecal and cecal content after 9 weeks of CD or HFD.
Figure 3: Body weight gain (n=37-40/group) in mice fed with control diet (CD) or high-fat diet (HFD) and inoculated with *B. wadsworthia* (Bw+) and treated with *L. rhamnosus* CNCM I-3690 (Lr+).
Figure 4: Blood glucose in mice fed with control diet (CD) or high-fat diet (HFD) and inoculated with *B. wadsworthia* (Bw+) and treated with *L. rhamnosus* CNCM I-3690 (Lr+).
Figure 5: Insulin in mice fed with control diet (CD) or high-fat diet (HFD) and inoculated with *B*. *wadsworthia* (Bw+) and treated with *L. rhamnosus* CNCM I-3690 (Lr+).
Figure 6: Homeostatic model assessment-insulin resistance (HOMA-IR) after 6h of fasting in mice fed with control diet (CD) or high-fat diet (HFD) and inoculated with *B. wadsworthia* (Bw+) and treated with *L. rhamnosus* CNCM I-3690 (Lr+).
Figure 7: Blood glucose level before and after oral glucose tolerance challenge (OGGT; 2g/kg mouse; n=27-40/group) in mice fed with control diet (CD) or high-fat diet (HFD) and inoculated with *B. wadsworthia* (Bw+) and treated with *L. rhamnosus* CNCM I-3690 (Lr+).
Figure 8: Area under the curve (AUC) of OGGT in mice fed with control diet (CD) or high-fat diet (HFD) and inoculated with *B. wadsworthia* (Bw+) and treated with *L. rhamnosus* CNCM I-3690 (Lr+).
Figure 9: Lipid area, calculated as % area of interest (AOI), in liver cross-sections stained with H&E in mice fed with control diet (CD) or high-fat diet (HFD) and inoculated with *B. wadsworthia* (Bw+) and treated with *L. rhamnosus* CNCM I-3690 (Lr+).
Figure 10: Representative pictures of liver stained with H&E in mice fed with control diet (CD) or high-fat diet (HFD) and inoculated with *B. wadsworthia* (Bw+) and treated with *L. rhamnosus* CNCM I-3690 (Lr+).
Figure 11: Liver triglycerides after 6h of food deprivation in mice fed with control diet (CD) or high-fat diet (HFD) and inoculated with *B. wadsworthia* (Bw+) and treated with *L. rhamnosus* CNCM I-3690 (Lr+).
Figure 12: Spearman correlation of fasting glucose and *B. wadsworthia* load in the cecal content. Statistical comparison was performed by first testing normality using Kolmogorov-Smirnov test and then ANOVA or Kruskal-Wallis test with Bonferroni or Dunn's *post hoc* test in mice fed with control diet (CD) or high-fat diet (HFD) and inoculated with *B. wadsworthia* (Bw+) and treated with *L. rhamnosus* CNCM I-3690 (Lr+).
Figures 13-17. *B. wadsworthia* augments HFD-induced bile acid dysmetabolism.

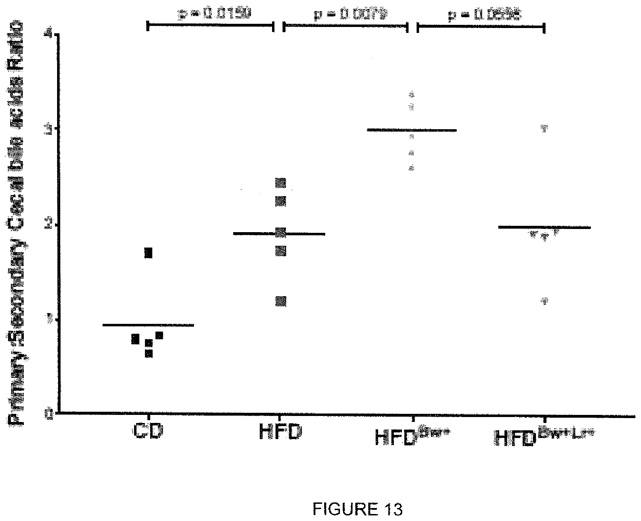

Figure 13: Ratio of primary to secondary bile acids in cecum in mice fed with control diet (CD) or high-fat diet (HFD) and inoculated with B. wadsworthia (Bw+) and treated with *L. rhamnosus* CNCM I-3690 (Lr+).

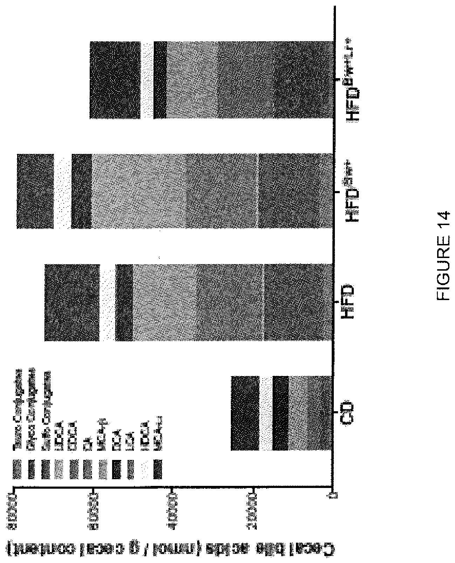

Figure 14: Stacked bar showing the bile acids concentration in the cecum in mice fed with control diet (CD) or high-fat diet (HFD) and inoculated with *B. wadsworthia* (Bw+) and treated with *L. rhamnosus* CNCM I-3690 (Lr+).

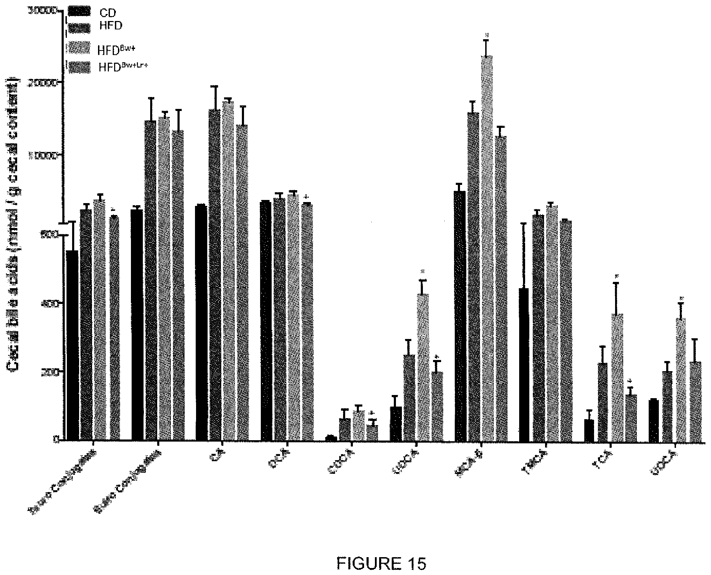

Figure 15: Concentration of difference bile acids in the cecum. (**p*<0.05 vs HFD-ASF, ⁺*p*<0.05 vs HFD-ASF^{Bw+}; n=5-6/group) in mice fed with control diet (CD) or high-fat diet (HFD) and inoculated with *B. wadsworthia* (Bw+) and treated with *L. rhamnosus* CNCM I-3690 (Lr+).

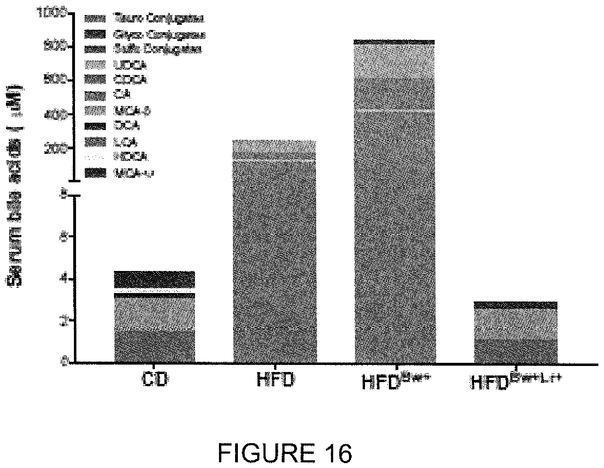

Figure 16: Stacked bar showing the bile acids concentration in the serum in mice fed with control diet (CD) or high-fat diet (HFD) and inoculated with *B. wadsworthia* (Bw+) and treated with *L*. *rhamnosus* CNCM I-3690 (Lr+).

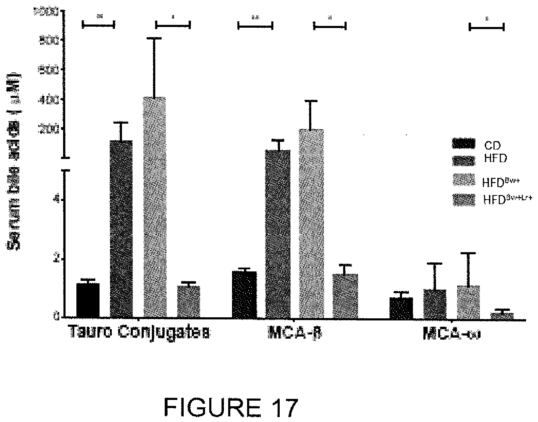

Figure 17: Concentration of difference bile acids in the serum (**p*<0.05, ***p*<0.05; n=5-6/group). Statistical comparison was performed by first testing normality using Kolmogorov-Smirnov test and then ANOVA or Kruskal-Wallis test with Bonferroni or Dunn's *post hoc* test in mice fed with control diet (CD) or high-fat diet (HFD) and inoculated with *B. wadsworthia* (Bw+) and treated with *L. rhamnosus* CNCM I-3690 (Lr+).

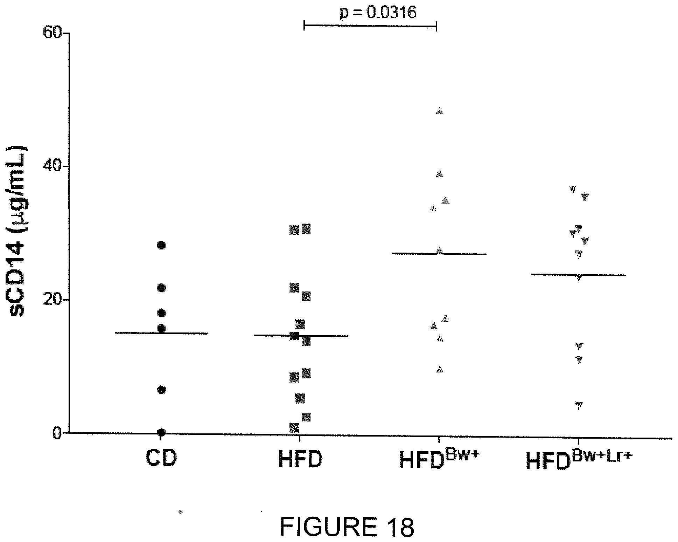

Figures 18-25 *B. wadsworthia* potentiates HFD-induced intestinal barrier dysfunction and inflammation.
Figure 18: Soluble CD14 (sCD14) in the serum in mice fed with control diet (CD) or high-fat diet (HFD) and inoculated with *B. wadsworthia* (Bw+) and treated with *L. rhamnosus* CNCM I-3690 (Lr+).

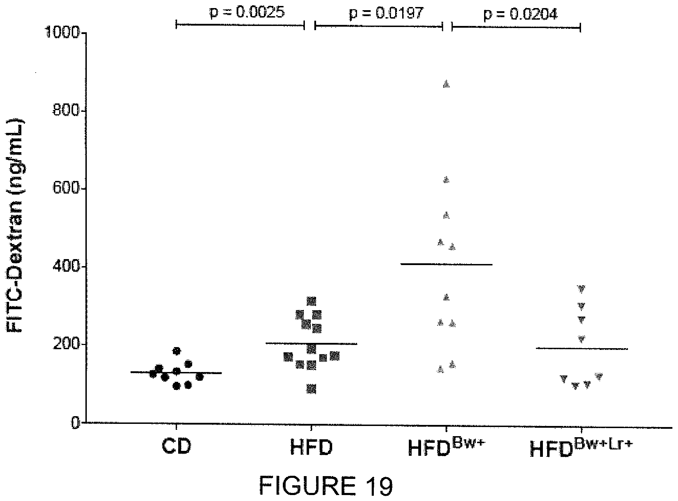

Figure 19: Concentration of FITC-dextran in the plasma 3h post-gavage in mice fed with control diet (CD) or high-fat diet (HFD) and inoculated with *B. wadsworthia* (Bw+) and treated with *L*. *rhamnosus* CNCM I-3690 (Lr+).

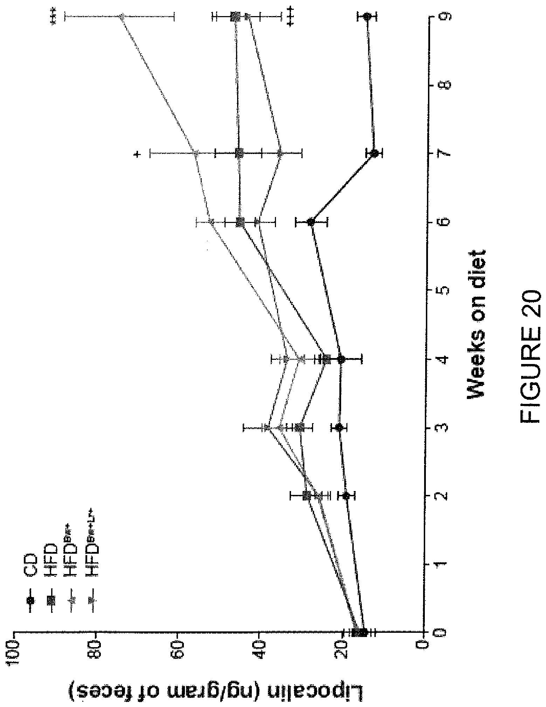

Figure 20: Concentration of lipocalin in the feces in mice fed with control diet (CD) or high-fat diet (HFD) and inoculated with *B. wadsworthia* (Bw+) and treated with *L. rhamnosus* CNCM I-3690 (Lr+).

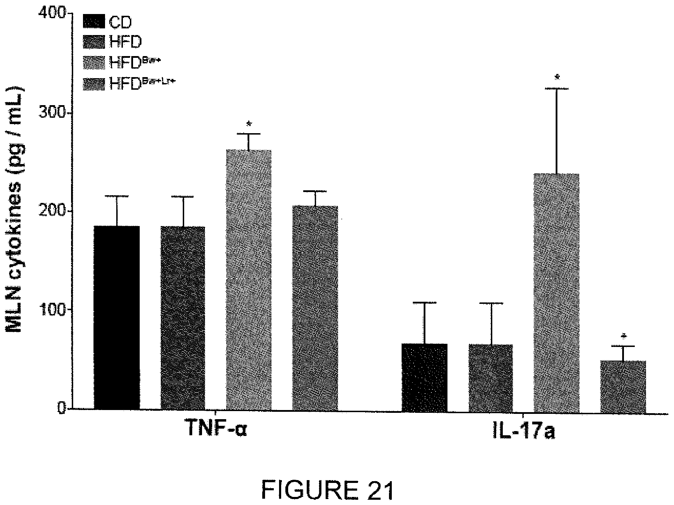

Figure 21: Cytokine production of mesenteric lymph node cells after 48h stimulation with PMA-ionomycin in mice fed with control diet (CD) or high-fat diet (HFD) and inoculated with *B*. *wadsworthia* (Bw+) and treated with *L. rhamnosus* CNCM I-3690 (Lr+).

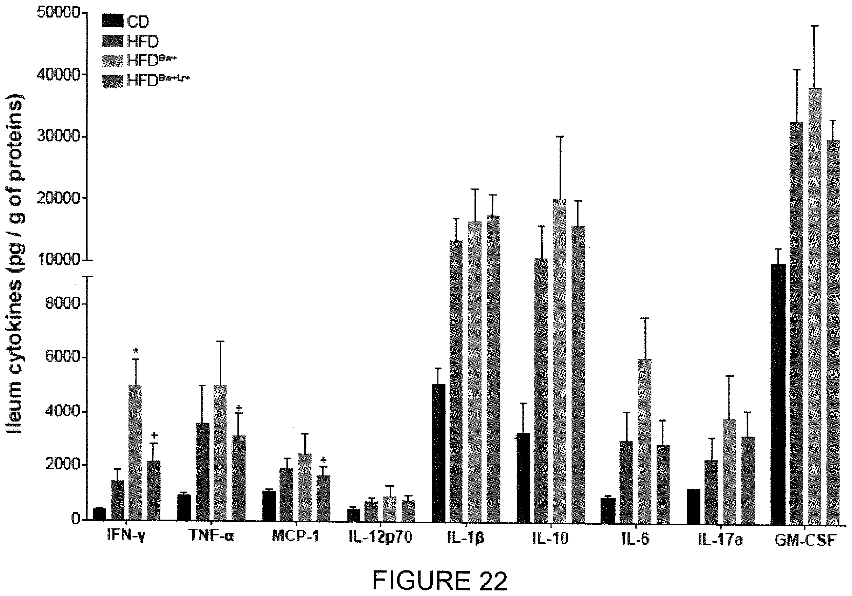

Figure 22: Cytokines from ilealhomogenates in mice fed with control diet (CD) or high-fat diet (HFD) and inoculated with *B. wadsworthia* (Bw+) and treated with *L. rhamnosus* CNCM I-3690 (Lr+).

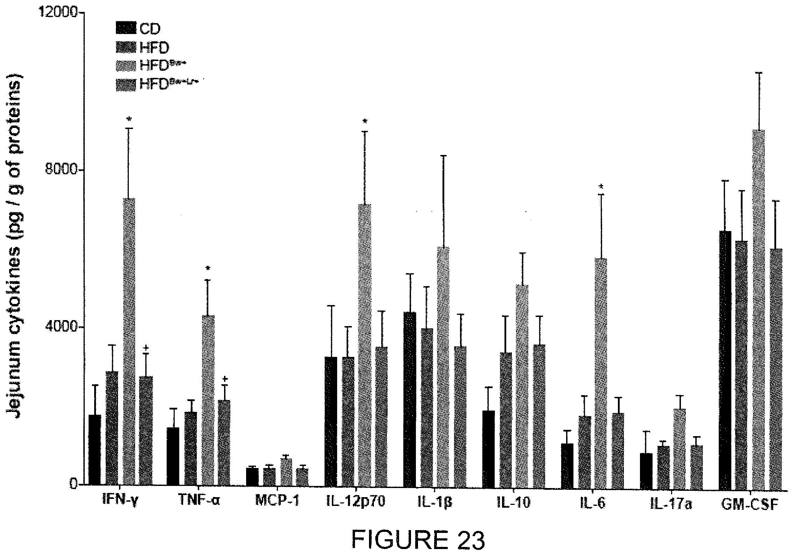

Figure 23: Cytokines from jejunal homogenates in mice fed with control diet (CD) or high-fat diet (HFD) and inoculated with *B. wadsworthia* (Bw+) and treated with *L. rhamnosus* CNCM I-3690 (Lr+).

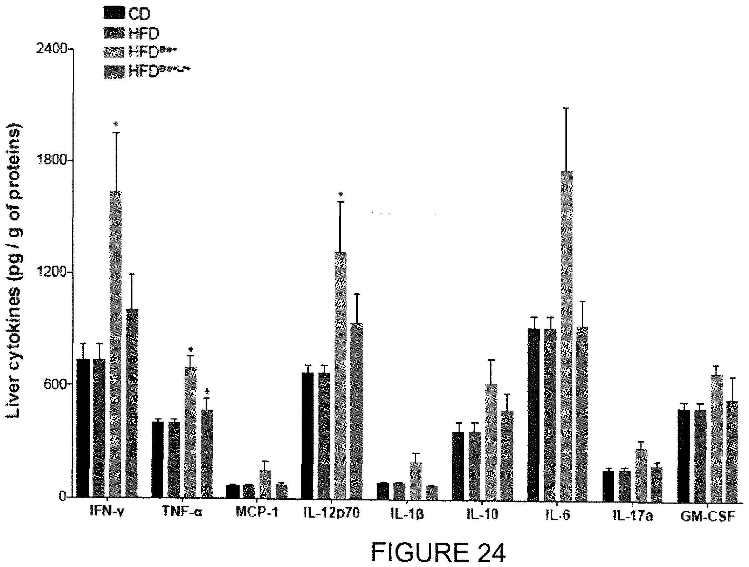

Figure 24: Cytokines from liver homogenates in mice fed with control diet (CD) or high-fat diet (HFD) and inoculated with *B. wadsworthia* (Bw+) and treated with *L. rhamnosus* CNCM I-3690 (Lr+).

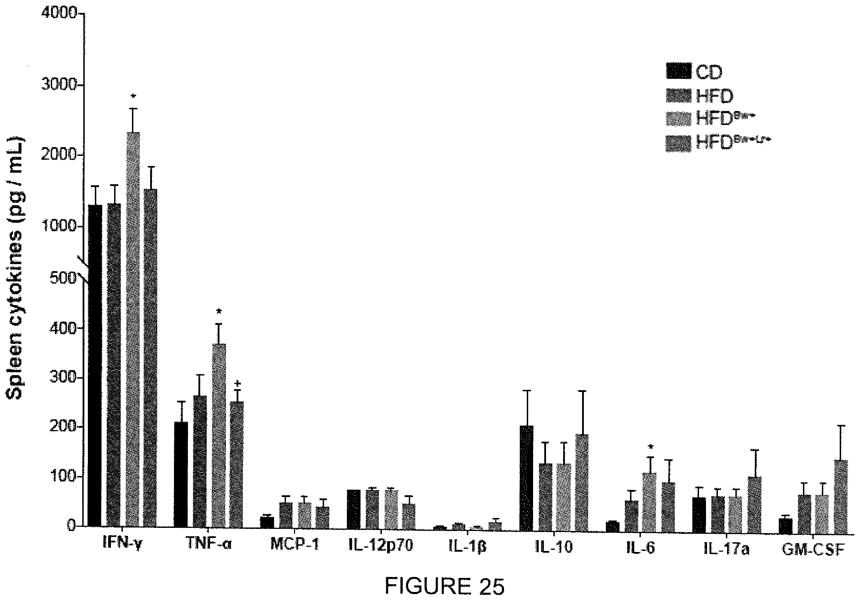

Figure 25: Cytokine production of splenic cells after 48h stimulation with PMA-ionomycin. Statistical comparison was performed by first testing normality using Kolmogorov-Smirnov test and then ANOVA or Kruskal-Wallis test with Bonferroni or Dunn's *post hoc* test (**p*-value vs HFD, ⁺*p*-value vs HFD^{Bw+}; n=6-16/group) in mice fed with control diet (CD) or high-fat diet (HFD) and inoculated with *B. wadsworthia* (Bw+) and treated with *L. rhamnosus* CNCM I-3690 (Lr+).

### Examples

### Animal and Study Design

For conventional experiment, male C57BL/6J mice were purchased from Janvier (France) and used after 1 week of receipt. Mice at 5 weeks of age were fed ad libitum with purified control diet (CD, Envigo MD.120508) or high fat diet (HFD, 18% milk-fat, Envigo MD.97222) or for 9 weeks. For deliberate *B. wadsworthia* inoculation, after maintaining the mice in HFD or CD for 2 weeks, mice were inoculated via oral gavage with ~10⁷ CFU of *B. wadsworthia* ATCC 49260 suspended in 200 µl of medium (Bacteroides bile esculin with 1% Taurine and 0.5 mg/ml cysteine) or medium alone for 3 consecutive days. For L. rhamnosus CNCM I-3690 treatment, 1 week after the last *B. wadsworthia* inoculation, mice were gavaged daily with 10⁹ CFU of L. rhamnosus CNCM I-3690 suspended in 200 µl of vehicle (phosphate buffer saline with 15% glycerol) or vehicle for 5 weeks. For cyclosporine experiment, 1 week after the last *B*. *wadsworthia* inoculation, mice were injected i.p. with ciclosporine (25 mg/kg; Sandimmum Novartis) or vehicle (PBS) 3x a week for 5 weeks.

For altered Schaedler flora (ASF) experiment, male C57BL/6J germ-free (GF) mice were obtained from Transgenese et Archivage Animaux Modeles (CNRS, UPS44, Orleans, France) and used after 1 week of receipt. Sterility was confirmed microscopically and by microbiological technique. ASF colonized mice were kindly provided by E. Verdu from McMaster University (Canada). Fresh cecal samples from ASF-colonized mice were suspended and diluted in prereduced sterile 0.9% NaCl with 15% glycerol (1 g in 10 ml) under anaerobic condition. Aliquots of ASF cecal suspension were stored at -80°C. GF mice (5 weeks of age) were inoculated via oral gavage with 200 µl of ASF cecal suspension and maintained on either HFD or CD. 3 weeks after ASF inoculation, mice were orally gavaged with *B. wadsworthia* or medium for 3 consecutive days. 1 week after the last *B. wadsworthia* inoculation, mice were gavaged daily with 10⁹ CFU of L. rhamnosus CNCM I-3690 or vehicle for 4 weeks.

Weekly food consumption was measured cage-wise. Mice were fasted for 6 hours prior to sacrifice and then put to sleep using isoflurane. Mice were culled by cervical dislocation and appropriate tissues were harvested. All experiments were performed in accordance with the Comite d'Ethique en Experimentation Animale.

### Oral glucose tolerance test

Oral glucose tolerance test was performed 3-5 days before the sacrifice. Mice were fasted by removing the food and bedding 1 hour before the onset of light cycle. After 6 hours of fasting, glucose solution (2 g/kg) was administered by oral gavage. Blood glucose level at time 0 (fasting glucose, taken before glucose gavage) and at 15, 30, 60 and 120 minutes after glucose gavage was analyzed using OneTouch glucometer (Roche). Glucose level was plotted against time and areas under the glucose curve (AUC) were calculated by following trapezoidal rule. Plasma insulin concentration (collected in EDTA-coated tubes) at time 0 (fasting insulin) and 30 min was analyzed from tail vein blood (collected in EDTA-coated tubes) using ultra sensitive mouse insulin ELISA kit (Alpco). Homeostatic model assessment of insulin resistance (HOMA-IR) was calculated according to the formula: fasting glucose (nmol/L) x fasting insulin (microU/L)/22.5.

### Measurements of plasma parameters

Blood samples were collected in heparin-coated tubes via cardiac puncture, centrifuged and then plasma samples were stored at -80°C. Plasma cholesterol, triglycerides, high-density lipoprotein (HDL), aspartate transaminase (AST) and alanine transaminase (ALT) measurement were performed by the Plateforme de Biochimie (CRI, UMR 1149, Paris) using Olympus AU400 Chemistry Analyzer.

### Measurements of bile acids

Measurement of bile acids (BA) composition and concentration in plasma and intestinal contents was performed by the Chemistry department at Saint Antoine Hospital (UMR 7203, France) using high performance liquid chromatography (HPLC, Agilent 1100, France) coupled in series with mass spectrometer (QTRAP 2000, Canada), as previously described.

### Measurements of SCFA

Measurement of the short-chain fatty acids (SCFA) from fecal content was performed by the mass spectrometer platform at Universite de Nantes (IRS-UN, France) using gas chromatography coupled with mass spectrometry, as previously described.

### Quantification of cytokines

Single cell suspensions from mesenteric lymph node (MLN) and spleen were isolated by smashing the cells in 70 µm mesh. 1 × 106 cells were plated in 24 well-plate and then stimulated with phorbol 12-myristate 13-acetate (PMA, 50ng/mL; Sigma-Aldrich) and ionomycin (1 uM; Sigma Aldrich) for 48h at 37°C. Supernatants were collected and used for cytokine analysis.

50 mg of intestinal tissues and liver samples were suspended in T-PER Tissue Protein Extraction Reagent (Thermo Scientific) and homogenized suing FastPrep (6 m/s in 40s). Homogenates were centrifuged and supernatants were used for cytokine and total protein concentration analysis. Total protein concentration of the tissue homogenates were analyzed using Pierce BCA Protein Assay Kit (Thermo Scientific). Cytokine concentrations were normalized according to the measured protein concentration.

Cytokines were measured using Legendplex Mouse Inflammation Panel (Biolegend) or individual ELISA kit (R&D Mouse DuoSet IL-6; Mabtech IFN-y, IL-17a ELISA kits; Ebioscience TNF-α ELISA kit).

### Liver histology and hepatic triglycerides measurement

A slice of left lobe of the liver was fixed in 4% PFA for 48 h and then transferred to ethanol, fixed in paraffin, trimmed, processed, sectioned into slices approximately 3 µm thick, mounted on a glass slide and stained with hemataoxylin and eosin (H&E). Hepatic lipids were evaluated and quantified as previously described.

### In-vivo intestinal permeability and plasma sCD14 measurement

In-vivo assay of intestinal barrier function was performed using fluorescein-conjugated dextran (FITC-dextran, 3-5kDA) method, as previously described (Martin et al, 2015). Briefly, on the day of sacrifice, FITC-dextran (0.6 mg/g of body weight) was administered to the mice by oral gavage and 3h later, blood samples were collected in heparin-coated tubes. Fluorescence intensity was measured in the plasma using a microplate reader (Tecan). Plasma concentration of soluble CD14 (sCD14) was measured using CD14 ELISA kit (R&D).

### Quantification of fecal LCN2

Frozen fecal samples were weighed and reconstituted in cold PBS. Samples were then agitated on a FastPrep bead beater machine for 40 s at setting 6 using 4.5 mm glass beads to obtain homogenous fecal suspension. Samples were then centrifuged for 5 min at 10,000g (4OC) and clear supernatants were collected and stored at --20°C until analysis. LCN2 levels were estimated using Duoset murine LCN2 Elisa Kit (R&D) as per manufacturer's instructions and expressed as pg / mg of stool.

### B. wadsworthia aggravates High Fat Diet-induced host metabolic impairment

To determine the consequence of *B. wadsworthia* abundance on host metabolic status, metabolic parameters were evaluated in mice after a period of high fat diet (HFD) feeding. HFDBw+ mice showed higher fasting glucose. Furthermore, significant increase in serum concentrations of aspartate transaminase (AST) and alanine transaminase (ALT) were observed in all HFD groups compared to chow diet (CD) fed mice, but there was no significant difference between HFD and HFDBw+ groups. Analysis of liver histology revealed that hepatic lipid content was significantly increased in HFDBw+ mice. In parallel, total hepatic triglyceride was significantly higher in HFDBw+ group than HFD, suggesting that *B. wadsworthia* have detrimental effects on this metabolic feature. All HFD-fed mice, regardless of treatment, had significantly elevated levels of total cholesterol and HDL in the plasma. Finally, a strong positive correlation between fasting glucose and *B. wadsworthia* load in the cecum was observed. Taken together, these results showed that the high abundance of *B. wadsworthia* potentiates specific HFD-induced host metabolic syndrome, with notable dysregulation of glucose homeostasis and liver function.

Preventing the over-abundance of *B. wadsworthia* in HFD reverts *B. wadsworthia-*associated metabolic dysfunctions

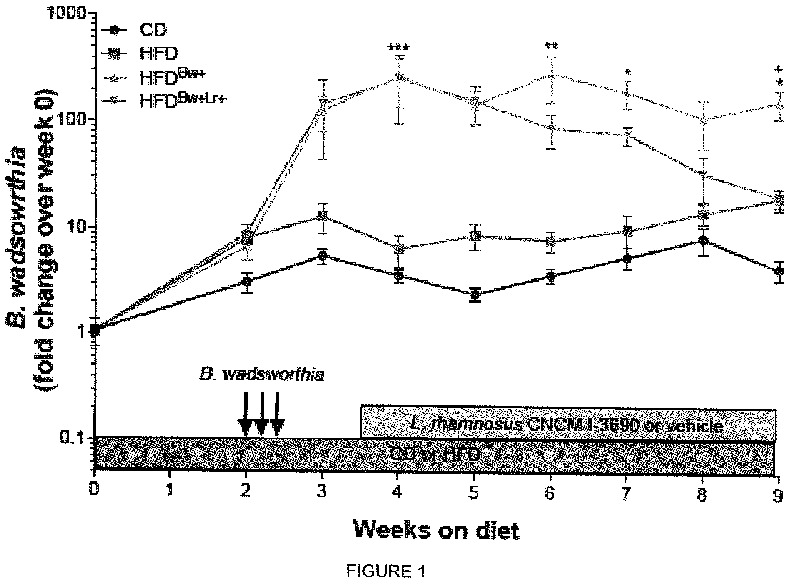

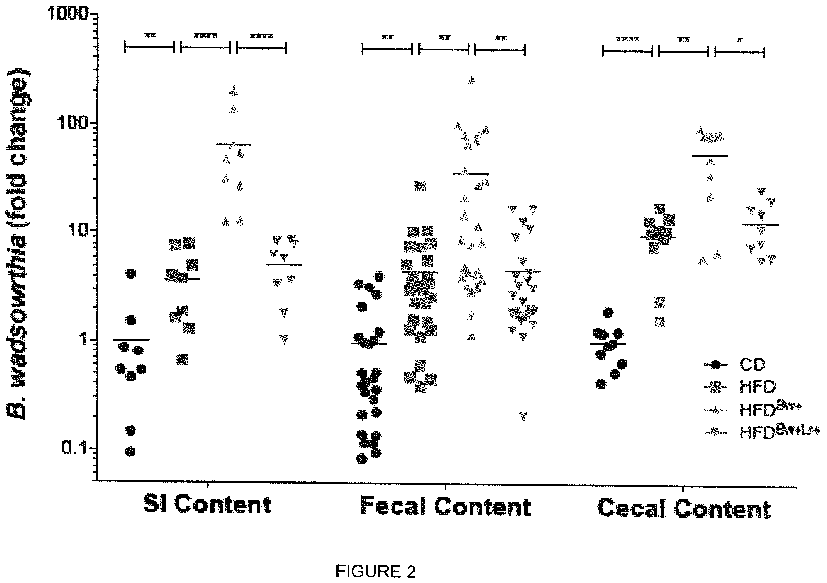

The inventors tested the ability of the *L. rhamnosus* CNCM I-3690 strain in the above-mentioned model. Daily oral gavage *of L. rhamnosus* CNCM I-3690 (Lr) induced a significant decrease in fecal B. wadsworthia load (Figure 1). Similarly, *L. rhamnosus* CNCM I-3690 was able to further reduce *B. wadsworthia* expansion in cecum and small intestine (Figure 2).

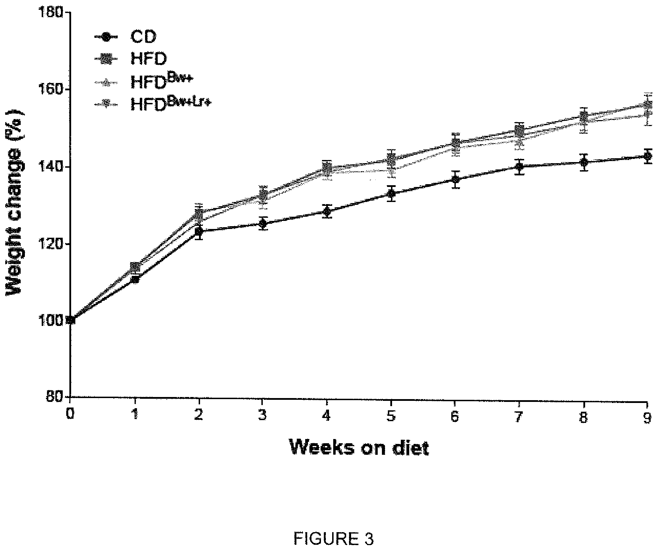

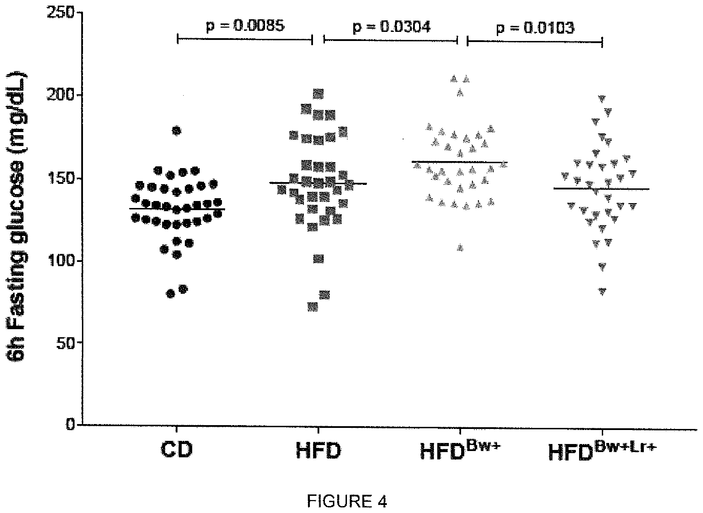

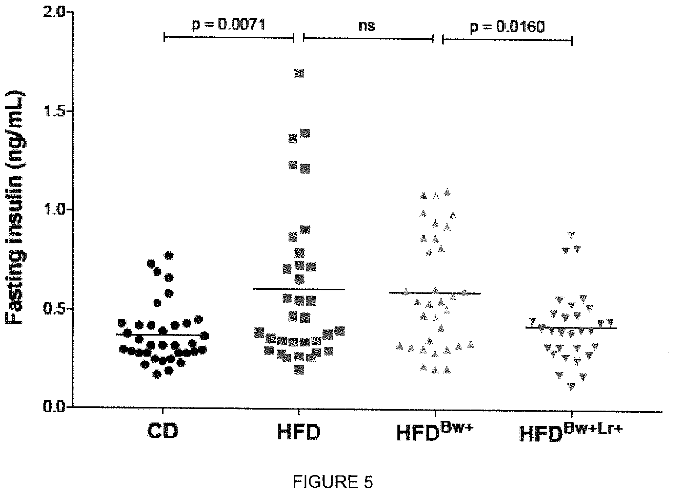

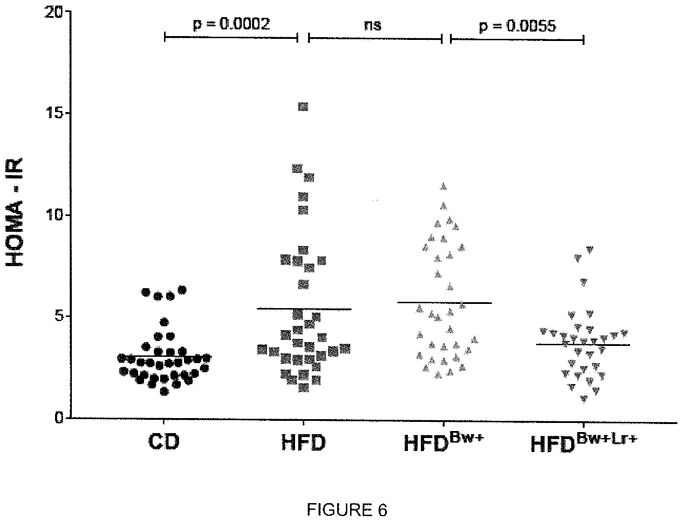

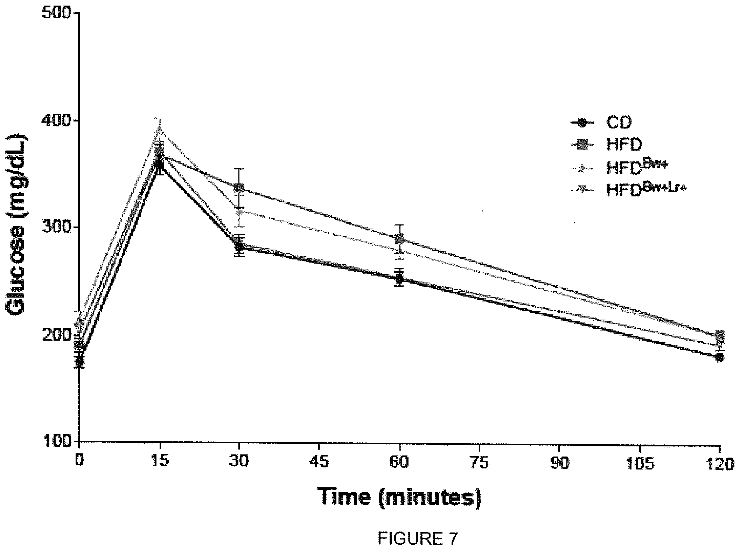

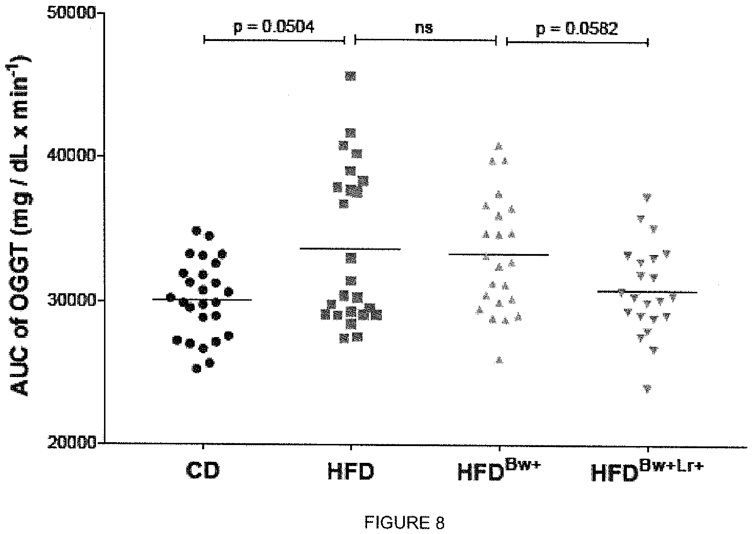

*L. rhamnosus* treated HFDBw+ mice (HFDBw+Lr+) showed reduced fasting glucose level, plasma insulin and HOMA-IR response (Figures 3-6). OGGT further revealed that HFDBw+Lr+ mice tended to control glucose level better than HFDBw+ (Figures 7-8). *L. rhamnosus* CNCM I-3690 also corrected the effect of HFD on insulin level (S4B)

### B. wadsworthia further enhances High Fat Diet-induced bile acid dysmetabolism

Host transcriptomic data revealed that *B. wadsworthia* modulates a number of genes involved in taurine metabolism, which is linked with bile acid homeostasis. Bile acids are increasingly recognized as important signaling factors and regulators of metabolism. As such, the inventors investigated the bile acid profile of mice harboring complex microbiota. The inventors found that HFD feeding leads to changes in bile acid composition in the cecum characterized by significantly higher total bile acids and elevated primary bile acids conjugates as opposed to secondary conjugates and decreased proportion of bile acids such as DCA and HDCA (Figures 13-15). *B. wadsworthia* tends to further dysregulate bile acid composition in the cecum with higher levels of taurocholic acid (TCA), a taurine-conjugated bile acid, as well as other bile acids such as UDCA and MCA-β. Furthermore, in the serum of HFD-fed mice, taurine conjugated bile acid concentration was more than 100-fold higher compared to CD, with an even stronger increase in HFDBw+ group (Figures 16-17). In contrast, HFDBw+Lr+ showed lower total and taurine-conjugated bile acids compared to HFDBw+, suggesting the efficiency of *L*. *rhamnosus* CNCM I-3690 to reverse the effect of HFD and *B. wadsworthia* on bile acids.

*B. wadsworthia* induces intestinal barrier dysfunction and amplifies HFD-driven inflammation that can be reverted by *L. rhamnosus* CNCM I-3690.

Based on the inventors simplified microbiota studies, the presence of *B. wadsworthia* up-regulated the global synthesis of LPS by the intestinal microbial communities and was further associated with higher systemic LPS. Guided by these results, the inventors similarly assessed the LPS availability in the systemic compartment in a HFD conventional mice model. In accordance with the results obtained from ASF-colonized mice, serum sCD14 level was significantly higher in HFDBw+ than in HFD mice (Figure 18). *L. rhamnosus* CNCM I-3690 ameliorated this phenotype.

Intestinal barrier dysfunction is an important feature in obesity and metabolic syndrome. The inventors hypothesized that this parameter may underlie the increased systemic bioavailability of LPS. Thus, the inventors assessed intestinal permeability using a classical permeability marker FITC-dextran. HFDBw+ mice exhibited increased intestinal permeability as demonstrated by higher serum FITC-dextran levels following oral gavage (Figure 19). This phenotype was reduced by *L. rhamnosus* CNCM I-3690. Overall, these results show that the increased *B. wadsworthia* abundance augments the impact of HFD-induced gut barrier alterations and *L. rhamnosus* CNCM I-3690 partially reverses this effect.

Disruption of the gut barrier may allow increased intestinal permeability to bacterial endotoxins, such as LPS, and in turn may increase mucosal inflammation and lead to systemic inflammation. Hence, the inventors next examined whether *B. wadsworthia* further exacerbates HFD-induced inflammatory response in conventional mice. The inventors first characterized the state of mucosal inflammation by quantifying lipocalin levels in the feces on different time-points during the duration of experiment (Figure 20). HFD feeding tended to show higher levels of lipocalin in the feces compared to CD but this was further and significantly increased in HFDBw+ mice, particularly at week 7 and 9. Cytokine levels in MLN, ileum and jejunum of HFDBw+ were similarly higher compared to either CD or HFD or both groups, underscoring a state of heightened mucosal immune response in HFDBw+ group (Figures 21-23). *L. rhamnosus* CNCM I-3690 treatment was able to dampen some of these responses, particularly for fecal lipocalin levels, TNF-α and IFN-γ (Figures 20-23).

The inventors further assessed the state of systemic inflammation and observed a similar pattern with significantly increased production of several pro-inflammatory cytokines such as IFN-y, TNF-α and IL-6 in the spleen and liver of HFDBw+ mice (Figures 24-25). Similar to mucosal immune response, *L. rhamnosus* CNCM I-3690 administration globally corrected *B*. *wadsworthia-*induced systemic inflammation. Taken together, these results showed that *B*. *wadsworthia* synergizes with HFD in inducing higher states of systemic and mucosal inflammation, which can be at least partly reversed by *L. rhamnosus* CNCM I-3690.

### DISCUSSION

High-saturated fat diets (HFD) were consistently associated with increase abundance of *B*. *wadsworthia,* a bacterium implicated in increase colitis severity of il-107- mice. However, the impact of *B. wadsworthia* on non-genetically susceptible host, and whether and how its expansion could promote an impaired metabolic function remains poorly understood. Here, the inventors utilized a hypothesis-driven approach, to dissect how *B. wadsworthia* is able to modulate host metabolic response to HFD. The inventors then tested the hypothesis in conventional HFD murine model. The results showed that, beside intestinal pro-inflammatory effects, *B. wadsworthia* promotes intestinal barrier defect, systemic inflammation, bile acid dysmetabolism and changes in microbiome functional profile, leading to the worsening of HFD-induced metabolic effects. Moreover, the inventors showed that *L. rhamnosus* CNCM I-3960 was able to reverse the majority of *B. wadsworthia-*driven host metabolic and inflammatory impairments.

*B. wadsworthia* had been previously shown to expand in the presence of taurine conjugated bile acids, especially taurocholic acid (TCA). Similarly, the inventors observed that *B*. *wadsworthia* grow in-vitro 1 log colony forming units more in the presence of taurine (1%). Thus, in conjunction with previous results, this suggests that taurine and its derivatives, particularly TCA, may not be necessary for *B. wadsworthia*'s survival but it is essential for its increased fitness and growth.

Bile acids are synthesized from cholesterol. In the liver, taurine, along with glycine, are used to conjugate bile acids to produce primary bile acids. Bile acids undergo enterohepatic circulation, which includes circulating in the intestine where primary bile acids are deconjugated and converted into secondary bile acids by the microbiota. Saturated animal-derived fats had been previously shown to promote the production of tauro-conjugated bile acids, such as TCA. The inventors showed that HFD significantly up-regulates genes involved in taurine metabolism with increased concentration of taurine conjugated bile acids and decreased proportion of secondary bile acids. *B. wadsworthia* further deregulates the bile acid disproportion in HFD context and this can be reversed by *L. rhamnosus* CNCM I-3690 treatment. Secondary bile acids have an important negative feedback role in decreasing bile acid synthesis; hence, the increased total serum and cecum bile acids in HFDBw+ group may be compounded by the decreased proportion of secondary bile acids. Additionally, unlike conjugated bile acids, unconjugated bile acids, such as cholic acid and chenodeoxycholic acid, are strong agonist for bile acid receptors such as Farnesoid X receptor and transmembrane G protein-coupled receptor. Signaling through these receptors activates transcriptional networks and signaling cascades relevant for cholesterol and lipid metabolism, maintenance of glucose and hepatic homeostasis, as well as genes involved in suppressing inflammation and strengthening intestinal barrier function. Similarly, previous studies have shown the pro-inflammatory properties of primary bile acids. Taken together, this suggest that *B. wadsworthia's* impact on bile acid metabolism may underlie the mechanism by which the bacterium potentiates HFD-induced metabolic impairment and host dysfunctions, particularly inflammation and barrier dysfunction.

To further determine the mechanistic basis by which *B. wadsworthia* impacts host metabolism and how *L. rhamnosus* CNCM I-3690 modulate these effects, the inventors performed transcriptomic analysis on both the host and microbiota. To fully understand the system, the inventors chose to work in a controlled microbiota environment, wherein bacterial and host function can be inferred to a specific microbe or condition. One of the key findings from said metatranscriptomics studies revealed that LPS synthesis pathway is highly up-regulated in HFDBw+ mice microbiota. This was paralleled by higher LPS translocation, which may at least partly explain the increased systemic inflammatory response the inventors observed in HFDBw+, both in ASF-colonized and conventional mice. *L. rhamnosus* CNCM I-3690 may be modulating the pro-inflammatory phenotype in HFDBw+ mice by decreasing the abundance of *B*. *wadsworthia* and/or through its intrinsic anti-inflammatory effects.

In addition to LPS synthesis, the presence of *B. wadsworthia* induced a decreased expression of microbial genes involved in butanoate metabolism in ASF-colonized mice. Furthermore, the decreased production of butyrate was confirmed by dosage in colon lumen. Aside from its effect in modulating inflammatory response, butyrate had been shown to reverse the increased intestinal permeability by assembly of tight junctions. Furthermore, dietary supplementation with butyrate had been previously shown to have preventive and therapeutic benefits in animal model of obesity and insulin resistance.

## Claims

1. A composition comprising at least one *Lactobacillus rhamnosus* CNCM I-3690, for use in treating bile acid dysmetabolism.

2. The composition for use according to claim 1 wherein serum or cecal bile acids are reduced.

3. The composition for use according to claim 1 wherein tauro-conjugated bile acids are reduced.

4. The composition for use according to any of claims 1 to 3, wherein the treated individual has a non-vegetarian diet and/or a high fat diet.

5. The composition for use according to any of claims 1 to 4, wherein the composition is a food product.

6. The composition for use according to any of claims 1 to 5, wherein the composition is a fermented dairy product.

## Patentansprüche

1. Zusammensetzung, die mindestens einen *Lactobacillus rhamnosus* CNCM I-3690 umfasst, zur Verwendung bei der Behandlung von Gallensäuredysmetabolismus.

2. Zusammensetzung zur Verwendung nach Anspruch 1, wobei Serum- oder Blinddarmgallensäuren reduziert werden.

3. Zusammensetzung zur Verwendung nach Anspruch 1, wobei Tauro-konjugierte Gallensäuren reduziert werden.

4. Zusammensetzung zur Verwendung nach einem der Ansprüche 1 bis 3, wobei die behandelte Person eine nicht vegetarische Ernährung und/oder eine fettreiche Ernährung hat.

5. Zusammensetzung zur Verwendung nach einem der Ansprüche 1 bis 4, wobei es sich bei der Zusammensetzung um ein Lebensmittelprodukt handelt.

6. Zusammensetzung zur Verwendung nach einem der Ansprüche 1 bis 5, wobei es sich bei der Zusammensetzung um ein fermentiertes Milchprodukt handelt.

## Revendications

1. Composition comprenant au moins un *Lactobacillus rhamnosus* CNCM I-3690, pour utilisation dans le traitement du dysmétabolisme des acides biliaires.

2. Composition pour son utilisation selon la revendication 1, dans laquelle les acides biliaires sériques ou caecaux sont réduits.

3. Composition pour son utilisation selon la revendication 1, dans laquelle les acides biliaires tauro-conjugués sont réduits.

4. Composition pour son utilisation selon l'une quelconque des revendications 1 à 3, dans laquelle l'individu traité a un régime non végétarien et/ou un régime riche en graisses.

5. Composition pour son utilisation selon l'une quelconque des revendications 1 à 4, dans laquelle la composition est un produit alimentaire.

6. Composition pour son utilisation selon l'une quelconque des revendications 1 à 5, dans laquelle la composition est un produit laitier fermenté.
